# EUROPEAN PATENT APPLICATION

(11) **EP 2 308 463 A1**
(43) Date of publication of application: **13.04.2011**
(21) Application number: 09172801.4
(22) Date of filing: 12.10.2009
(51) Int. Cl.: A61K 9/00, A61K 31/167

(54) **Aqueous acetaminophen compositions and method of preparation**

(71) Applicant: EMP Pharma GmbH, 8002 Zürich (CH)
(72) Inventor: PSARRAKIS, Yannis, 19500, Lavrion - Attica (GR); LIOUMIS, Kosta, 19500, Lavrion - Attica (GR)
(74) Representative: Hendriksen, David

(57) **Abstract**

The invention relates to a_liquid composition suitable for parenteral administration, comprising at least 1 mg/ml of acetaminophen. The invention also relates to a method of preparation of such a composition. The liquid composition may comprise at least one pharmaceutically acceptable chelating agent and/or a tonicity agent and/or an antioxidant and/or a solubilising agent, and is suitable for intravenous injection and/or intramuscular injection.

## Description

### FIELD OF THE INVENTION

The invention relates to a liquid composition suitable for parenteral administration, comprising at least 1 mg/ml of acetaminophen. The invention also relates to a method of preparation of such a composition.

### BACKGROUND OF THE INVENTION

Acetaminophen, also known as paracetamol or its chemical name para-acetylaminophenol, is a well known pain killer. Acetaminophen is best known in solid administration forms, such as pressed tablets. However, oral administration routes shows a relatively slow uptake of the effective component. For some applications, parenteral administration is preferred. The achievable acetaminophen dose and uptake rates by parenteral administration are limited by the poor water solubility of acetaminophen.
A known formulation for intravenous administration by infusion at a dose of 10 mg/ml is known under the trade name Perfalgan, marketed by Bristol-Myers Squibb. This is an aqueous solution of acetaminophen.

### OBJECT AND SUMMARY OF THE INVENTION

It is an object of the invention to provide an improved acetaminophen liquid formulation suitable for parenteral administration. It is an object to provide liquid acetaminophen solutions having a sufficient acetaminophen stability. Another object of this invention is to enable high dosage acetaminophen formulations suitable for intramuscular administration. Liquid formulations comprising more than 10mg/ml are considered to be high dosage formulations

The invention provides a liquid composition suitable for parenteral administration, comprising at least 1 mg/ml of acetaminophen, preferably at least 10 mg/ml, most preferably at least 100 mg/ml, and at least one pharmaceutically acceptable excipient.

In a preferred embodiment, a liquid composition is suitable for parenteral administration, comprising at least 1 mg/ml of acetaminophen or a derivative thereof, and at least one pharmaceutically acceptable chelating agent. Preferably, the composition comprises at least 10 mg/ml acetaminophen, more preferably 100 mg/ml, most preferably at least 150 mg/ml.

The addition of a chelating agent was shown to slow down the rate of acetaminophen degradation in solutions. Acetaminophen degrades in liquid formulations over time at a significantly faster rate than in solid formulations such as tablets, and the invention aims to at least slow down the degradation rate in liquid aqueous formulations.

Chelating agents are chemical compounds that form stable soluble complexes (chelates) with traces of alkaline earth and heavy metal ions. It is postulated that chelation reduces or hampers catalytic processes that such ions may have in the degradation of acetaminophen. This significantly increases the shelf life of liquid formulations, i.e. the time a stored solution retains a certain percentage (for instance 90%) of the originally available active component. Also for freshly prepared formulations of acetaminophen, the addition of a chelating agent ensures the desired concentration of active acetaminophen species (as opposite to inactive degradation products) is preserved prior to administration, and is less susceptible to degradation by sources of potentially degrading metal surfaces, for instance the surface of metallic stirrer equipment or syringes. The reduction in degradation rate was found in aqueous solutions of acetaminophen, but also in other solvents or mixtures of solvents.

Preferably, the acetaminophen concentration in the composition is at least 1 mg/ml, preferably at least 10 mg/ml, most preferably at least 100 mg/ml. The achievable concentration is limited by the solubility of acetaminophen in the chosen solvent. The concentration may be in the range from 1-180 mg/ml. Preparations comprising very high and stable doses of (higher than 100 mg/ml) may be achieved using solubilizer agents and stabilizing agents according to the invention. Aqueous compositions based on dimethylacetamide , prepared using hot water, yield clear and stable formulations of acetaminophen, which may be further stabilized using suitable chelating agents, pH and/or antioxidants.

Preferably,_the chelating agent comprises at least one water-soluble chelating agent selected from the group consisting of malic acid, fumaric acid, tartaric acid, edetic acid, dipotassium edetate; disodium edetate, calcium disodium, monosodium edetate; trisodium edetate, ethylenediamine tetraacetic acid (EDTA), citric acid, anhydrous citric acid, sodium citrate dihydrate, maltol and/or ethyl maltol, or pharmaceutically acceptable salt derivatives thereof. These compounds have shown to be effective in slowing down the degradation rates of acetaminophen in liquid compositions. Pharmaceutically acceptable salt derivatives typically includes lithium, sodium, potassium, calcium and magnesium salts, including mixed salts.

In a preferred embodiment, the at least one water-soluble chelating agent comprises ethylenediamine tetraacetic acid (EDTA) or a pharmaceutically acceptable salt thereof. Ethylenediamine tetraacetic acid (EDTA) was shown to be particularly effective in slowing down degradation rates of acetaminophen. Pharmaceutically acceptable salt derivatives typically includes lithium, sodium, potassium, calcium and magnesium salts, including mixed salts of EDTA. A commercially available mixed salt is the calcium disodium salt of EDTA (Na₂Ca-EDTA). Other popular forms are the disodium salt and the tetrasodium salt. The composition comprising EDTA could also comprise other chelating agents described herein. Preferably, EDTA, calculated from the free acid, is added in amounts of at least 0.01 % w/v, preferably in the range of 0.01-0.1 w/v%.

It is preferred if the composition also comprises at least one tonicity agent selected from the group consisting of mannitol, sodium chloride and phosphate salts. Such an agent can be used to prevent or at least lower disadvantageous reactions such as irritations that a patients body may show due to parenteral administration. For intravenous administration, the concentration should have a tonicity compatible with human blood.

In a preferred embodiment the composition comprises a combination of mannitol and disodium phosphate. This combination shows a slower development of degradation products of acetaminophen than compositions of comparable tonicity using sodium chloride.

It is preferred if the composition also comprises at least one acetaminophen solubilising agent. This allows for relatively high concentrations of acetaminophen in solution, and at least partially prevents precipitation during processing and storage of the solution. The minimum effective amount of solubilising agent depends on the amount of acetaminophen to be dissolved in the composition.

In a preferred embodiment, the solubilising agent is selected from the group consisting of dimethylacetamide, glycerine, glycofurol, povidone, ethanol alcohol (95%) or admixtures thereof. Each of these solvents combines an excellent solubility for acetaminophen with a good acceptability for parenteral administration. Each of these can be mixed with water, but solutions may also be prepared without water. These excipients have very high solubility in water. The important is the ratio with between the excipient and acetaminophen. Also, I dont have figures for those excipients.

It is preferred if the solubilising agent comprises dimethylacetamide. Acetaminophen shows an excellent solubility in dimethylacetamide as well as dimethylacetamide/water mixtures. Preferably, the ratio of acetaminophen and dimethylacetaminde is at least 1:1 by weight, preferably in the range from 1:1 to 3:1. A combination of dimethylacetamide and glycofurol also shows excellent properties.

It is preferred if the composition also comprises at least one antioxidant. The addition of antioxidants prevents or at least slows down the degradation of acetaminophen in solution, and also allows for easier handling of the solution when exposed to air. An antioxidant for the present invention is defined as an agent that is oxidised easier than acetaminophen. The combination of at least one chelating agent and at least one antioxidant shows an even improved effect in preventing or at least slowing down the degradation of acetaminophen in solution when compared to the same solution including only an antioxidant or only a chelating agent. Preferably, the antioxidant is water-soluble.

Preferably, the at least one antioxidant is selected from the group of water-soluble antioxidants consisting of ascorbic acid, sodium bisulfite, erythorbic Acid, ascorbic acid, sodium erythorbate, ascorbyl palmitate; sodium ascorbate, ascorbyl palmitate, calcium ascorbate. sodium sulfite, sodium sulfite heptahydrate, sodium metabisulfite, Potassium metabisulfite, sodium bisulfite, propionic acid, sodium propionate, malic Acid, d-Malic acid, and I-Malic acid. Also, antioxidants having a low water-solubility may be used. Butylated hydroxyanisole (BHA), propyl gallate, butylated hydroxytoluene (BHT) and thymol are preferred. In order to achieve an effective amount of antioxidant having a low water-solubility, a solubility enhancing agent, preferably ethanol (alcohol 95%) would have to be used.

In a preferred embodiment, the at least one water-soluble antioxidant agent comprises sodium metabisulfite or a pharmaceutically acceptable salt thereof. Sodium metabisulfite achieved very good results in the stability studies. And works in satisfactory way as antioxidant for the preservation of acetaminophen.

In another preferred embodiment the at least one water-soluble antioxidant comprises thioglycol. This compounds shows is highly soluble in water, chemically stable without Incompatibilities with acetaminophen, and proved to be a relatively nontoxic and nonirritant material for parenteral usage. This antioxidant could be added in a concentration of at least 0.02% w/w.

It is preferred if the composition is an aqueous solution having a pH between 5 and 6, preferably having a pH in the range of 5.4-5.6. Within this pH range combined with a chelating compounds yields the slowest degradation of acetaminophen in solution.

In a preferred embodiment, the composition is suitable for intravenous injection. Such a solution is typically also be suitable for intramuscular injection.

In another preferred embodiment, the composition is suitable for intramuscular injection. Such a solution may be unsuitable for intravenous injection, due to the stricter requirements for intravenous injection, for instance regarding to tonicity and pH of the solution. For intramuscular injections, such limitations for the preventions of side effects are usually less strict.

The invention also provides a method for the preparation of a composition according to any of the preceding claims, comprising the steps of:
- dissolving at least 1 mg/ml of acetaminophen or a derivative thereof in a solvent, preferably water,
- and dissolving at least one pharmaceutically acceptable chelating agent in the solvent. Other components of the composition may be dissolved in the solution before, after or simultaneously during these steps.

Preferably, the solvent comprises dimethylacetamide or a mixture comprising dimethylacetamide and water.

The invention also provides a method for the preparation of a high dosage composition of acetaminophen according to any of the preceding claims, comprising the steps of:
- _dissolving at least 100 mg/ml, preferably at least 150 mg/ml of acetaminophen or a derivative thereof in hot water heated at 60C,
- the addition of a appropriate solubilising agent or solvent system, preferably dimethylacetamide, preferably in a ratio 1:1 , 2:1 or 3:1 1 with respect to the aqueous acetaminophen solution. This yields a stable, clear solution at room temperature with a very high concentration. This aqueous solution may optionally be further stabilized by adjusting the pH, adding at least one pharmaceutically acceptable chelating agent in the solvent and/ or adding an antioxidant

It is advantageous if at least during the dissolving step the solvent, preferably water, is heated to at least 60 °C, preferably approximately 70 °C. This allows for a relatively fast dissolution of acetaminophen.

### BRIEF DESCRIPTION OF THE DRAWINGS

### DESCRIPTION OF PREFERRED EMBODIMENTS

The following examples 1-16 are suitable for both intramuscular and intravenous application.

### Aqueous solutions with an additional solvent

The manufacturing process for the examples 1 □16_is:
A. Dissolve paracetamol (acetaminophen) using the solvent system, in water (in some cases use of hot water 70°C_or 60°C)
B. Add the rest of water
C. Add the rest of excipients
D. Adjust the pH value using HCL or NaOH 1N, if needed.
E. Fill the solution into suitable containers, which are sealed airtight for testing.

This method yields clear solutions of acetaminophen.
Solvent systems used in the examples are glycofurol, dimethylacetamide (DMAC), glycerine, povidone, 95% ethanol alcohol and mixtures thereof, typically used as a mix with water.

### EXAMPLE 1

| **INGREDIENTS** | **Quantity for 100 ml of_solution** |
|---|---|
| **Paracetamol** | 15 gr |
| **Glycofurol** | 45 gr |
| **Hydrochloric acid** | *qs to pH 5.50* |
| **Sodium hydroxide** | *qs to pH 5.50* |
| **Purified water** | qs to 100 ml |
| **TOTAL VOLUME** | **100 ml** |

### EXAMPLE 2

| **INGREDIENTS** | **Quantity for 100 ml of_solution** |
|---|---|
| **Paracetamol** | 15 gr |
| **Glycofurol** | 75 gr |
| **Hydrochloric acid** | *qs to pH 5.50* |
| **Sodium hydroxide** | *qs to pH 5.50* |
| **Purified water** | qs to 100 ml |
| **TOTAL VOLUME** | **100 ml** |

### EXAMPLE 3

| **INGREDIENTS** | **Quantity for 100 ml of_solution** |
|---|---|
| **Paracetamol** | 1 gr |
| **Glycofurol** | 3 gr |
| **Hydrochloric acid** | *qs to pH 5.50* |
| **Sodium hydroxide** | *qs to pH 5.50* |
| **Purified water** | qs to 100 ml |
| **TOTAL VOLUME** | **100 ml** |

### EXAMPLE 4

| **INGREDIENTS** | **Quantity for 100 ml of_solution** |
|---|---|
| **Paracetamol** | 1 gr |
| **DMAC** | 1 gr |
| **Hydrochloric acid** | *qs to pH 5.50* |
| **Sodium hydroxide** | *qs to pH 5.50* |
| **Purified water** | qs to 100 ml |
| **TOTAL VOLUME** | **100 ml** |

### EXAMPLE 5

| **INGREDIENTS** | **Quantity for 100 ml of_solution** |
|---|---|
| **Paracetamol** | 1 gr |
| **DMAC** | 5 gr |
| **Hydrochloric acid** | *qs to pH 5.50* |
| **Sodium hydroxide** | *qs to pH 5.50* |
| **Purified water** | qs to 100 ml |
| **TOTAL VOLUME** | **100 ml** |

### EXAMPLE 6

| **INGREDIENTS** | **Quantity for 100 ml of_solution** |
|---|---|
| **Paracetamol** | 1 gr |
| **DMAC** | 1.6 gr |
| **Hydrochloric acid** | *qs to pH 5.50* |
| **Sodium hydroxide** | *qs to pH 5.50* |
| **Purified water** | qs to 100 ml |
| **TOTAL VOLUME** | **100 ml** |

### EXAMPLE 7

| **INGREDIENTS** | **Quantity for 100 ml of_solution** |
|---|---|
| **Paracetamol** | 1 gr |
| **DMAC** | 0.5 gr |
| **Hydrochloric acid** | *qs to pH 5.50* |
| **Sodium hydroxide** | *qs to pH 5.50* |
| **Purified water** | qs to 100 ml |
| **TOTAL VOLUME** | **100 ml** |

### EXAMPLE 8

| **INGREDIENTS** | **Quantity for 100 ml of_solution** |
|---|---|
| **Paracetamol** | 15 gr |
| **DMAC** | 15 gr |
| **Purified water** | qs to 100 ml |
| **TOTAL VOLUME** | **100 ml** |

### EXAMPLE 8.1

| **INGREDIENTS** | **Quantity for 100 ml of_solution** |
|---|---|
| **Paracetamol** | 15 gr |
| **DMAC** | 30 gr |
| **Purified water** | qs to 100 ml |
| **TOTAL VOLUME** | **100 ml** |

### EXAMPLE 8.2

| **INGREDIENTS** | **Quantity for 100 ml of_solution** |
|---|---|
| **Paracetamol** | 15 gr |
| **DMAC** | 35 gr |
| **Purified water** | qs to 100 ml |
| **TOTAL VOLUME** | **100 ml** |

### EXAMPLE 8.3

| **INGREDIENTS** | **Quantity for 100 ml of_solution** |
|---|---|
| **Paracetamol** | 15 gr |
| **DMAC** | 50 gr |
| **Purified water** | qs to 100 ml |
| **TOTAL VOLUME** | **100 ml** |

### EXAMPLE 8.4

| **INGREDIENTS** | **Quantity for 100 ml of_solution** |
|---|---|
| **Paracetamol** | 18 gr |
| **DMAC** | 50 gr |
| **Purified water** | qs to 100 ml |
| **TOTAL VOLUME** | **100 ml** |

### EXAMPLE 8.5

| **INGREDIENTS** | **Quantity for 100 ml of_solution** |
|---|---|
| **Paracetamol** | 18 gr |
| **DMAC** | 20 gr |
| **Purified water** | qs to 100 ml |
| **TOTAL VOLUME** | **100 ml** |

### EXAMPLE 9

| **INGREDIENTS** | **Quantity for 100 ml of_solution** |
|---|---|
| **Paracetamol** | 15 gr |
| **DMAC** | 15 gr |
| **Hydrochloric acid** | *qs to pH 4* |
| **Sodium hydroxide** | *qs to pH 4* |
| **Purified water** | qs to 100 ml |
| **TOTAL VOLUME** | **100 ml** |

### EXAMPLE 9.1

| **INGREDIENTS** | **Quantity for 100 ml of_solution** |
|---|---|
| **Paracetamol** | 15 gr |
| **DMAC** | 30 gr |
| **Hydrochloric acid** | *qs to pH 4* |
| **Sodium hydroxide** | *qs to pH 4* |
| **Purified water** | qs to 100 ml |
| **TOTAL VOLUME** | **100 ml** |

### EXAMPLE 10

| **INGREDIENTS** | **Quantity for 100 ml of_solution** |
|---|---|
| **Paracetamol** | 1 gr |
| **DMAC** | 0.5 gr |
| **Alcohol_95°** | 2 gr |
| **Hydrochloric acid** | *qs to pH 5.50* |
| **Sodium hydroxide** | *qs to pH 5.50* |
| **Purified water** | qs to 100 ml |
| **TOTAL VOLUME** | **100 ml** |

### EXAMPLE 11

| **INGREDIENTS** | **Quantity for 100 ml of_solution** |
|---|---|
| **Paracetamol** | ∼1 gr |
| **Hydrochloric acid** | *qs to pH 5.50* |
| **Sodium hydroxide** | *qs to pH 5.50* |
| **water 0.9%*** | qs to 100 ml |
| **TOTAL VOLUME** | **100 ml** |

### EXAMPLE 12

| **INGREDIENTS** | **Quantity for 100 ml of_solution** |
|---|---|
| **Paracetamol** | ∼1 gr |
| **Glycerine** | 3 gr |
| **Hydrochloric acid** | *qs to pH 5.50* |
| **Sodium hydroxide** | *qs to pH 5.50* |
| **Purified water** | qs to 100 ml |
| **TOTAL VOLUME** | **100 ml** |

### EXAMPLE 13

| **INGREDIENTS** | **Quantity for 100 ml of_solution** |
|---|---|
| **Paracetamol** | ∼1 gr |
| **Glycerine** | 1 gr |
| **Alcohol 95°** | 1 gr |
| **Hydrochloric acid** | *qs to pH 5.50* |
| **Sodium hydroxide** | *qs to pH 5.50* |
| **Purified water** | qs to 100 ml |
| **TOTAL VOLUME** | **100 ml** |

### EXAMPLE 14

| **INGREDIENTS** | **Quantity for 100 ml of_solution** |
|---|---|
| **Paracetamol** | ∼1 gr |
| **Glycerine** | 1 gr |
| **Dimethylacetamide (DMAC)** | 1 gr |
| **Hydrochloric acid** | *qs to pH 5.50* |
| **Sodium hydroxide** | *qs to pH 5.50* |
| **Purified water** | qs to 100 ml |
| **TOTAL VOLUME** | **100 ml** |

### EXAMPLE 15

| **INGREDIENTS** | **Quantity for 100 ml of_solution** |
|---|---|
| **Paracetamol** | ∼1 gr |
| **Povidone** | 1 gr |
| **Hydrochloric acid** | *qs to pH 5.50* |
| **Sodium hydroxide** | *qs to pH 5.50* |
| **Purified water** | qs to 100 ml |
| **TOTAL VOLUME** | **100 ml** |

### EXAMPLE 16

| **INGREDIENTS** | **Quantity for 100 ml of_solution** |
|---|---|
| **Paracetamol** | ∼1 gr |
| **Povidone** | 2 gr |
| **Hydrochloric acid** | *qs to pH 5.50* |
| **Sodium hydroxide** | *qs to pH 5.50* |
| **Purified water** | qs to 100 ml |
| **TOTAL VOLUME** | **100 ml** |

### Aqueous solutions: Examples 17-33

The manufacturing process for the examples 17 □33_is:
A. Dissolve Paracetamol using the solvent system, in water (in some cases the water is heated to_70°C_or 60°C to speed up the dissolving proces)
B. Add the rest of water
C. Add the rest of excipients_(chelating agents or /and antioxidants)
D. Adjust the pH value using HCL or/and_NaOH 1N
E. Fill the solution into suitable containers
In case of alcohol usage of an excipient insoluble or poorly soluble in water the excipient is first dissolved in ethanol alcohol 95% before adding the excipient to the solution in Step C.

### EXAMPLE 17

| **INGREDIENTS** | **Quantity for 100 ml of_solution** |
|---|---|
| **Paracetamol** | _1*gr* |
| **Malic acid** | *0.02gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 17.1

| **INGREDIENTS** | **Quantity for 100 ml of_solution** |
|---|---|
| **Paracetamol** | _1*gr* |
| **Fumaric_acid** | *0.02gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 17.2

| **INGREDIENTS** | **Quantity for 100 ml of_solution** |
|---|---|
| **Paracetamol** | _1*gr* |
| **Tartaric acid** | *0.02gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 17.3

| **INGREDIENTS** | **Quantity for 100 ml of_solution** |
|---|---|
| **Paracetamol** | 1*gr* |
| **Citric acid** | *0.05gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 17.4

| **INGREDIENTS** | **Quantity for 100 ml of_solution** |
|---|---|
| **Paracetamol** | _1*gr* |
| **Ascorbyl palmitate** | *0.03gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 17.5

| **INGREDIENTS** | **Quantity for 100 ml of_solution** |
|---|---|
| **Paracetamol** | _1*gr* |
| **Maltol** | *0.01gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 18

| **INGREDIENTS** | **Quantity for 100 ml of_solution** |
|---|---|
| **Paracetamol** | _1*gr* |
| **Metabisulphite sodium** | *0.07gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 18.1

| **INGREDIENTS** | **Quantity for 100 ml of_solution** |
|---|---|
| **Paracetamol** | _1*gr* |
| **Sulfite sodium** | *0.05gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 18.2

| **INGREDIENTS** | **Quantity for 100 ml of_solution** |
|---|---|
| **Paracetamol** | _1*gr* |
| **Ascorbic acid** | *0.5gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 18.3

| **INGREDIENTS** | **Quantity for 100 ml of_solution** |
|---|---|
| **Paracetamol** | _1*gr* |
| **Sodium propionate** | *0.5gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 18.4

| **INGREDIENTS** | **Quantity for 100 ml of_solution** |
|---|---|
| **Paracetamol** | _1*gr* |
| **Erythorbic acid** | *0.5gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 18.5

| **INGREDIENTS** | **Quantity for 100 ml of_solution** |
|---|---|
| **Paracetamol** | _1*gr* |
| **Sodium erythorbate** | *0.5gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |
| **Final pH** | ∼5.5 |

### EXAMPLE 19

| **INGREDIENTS** | **Quantity for 100 ml of_solution** |
|---|---|
| **Paracetamol** | _1*gr* |
| **Edetate sodium** | *0.07gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 20

| **INGREDIENTS** | **Quantity for 100 ml of_solution** |
|---|---|
| **Paracetamol** | _1*gr* |
| **Monothioglycerol** | *0.3gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 21

| **INGREDIENTS** | **Quantity for 100 ml of_solution** |
|---|---|
| **Paracetamol** | _1*gr* |
| **Monothioglycerol** | *1gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 22

| **INGREDIENTS** | **Quantity for 100 ml of_solution** |
|---|---|
| **Paracetamol** | _1*gr* |
| **Dimethylacetamide (DMAC)** | *0.5gr* |
| **Malic acid** | *0.03gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 23

| **INGREDIENTS** | **Quantity for 100 ml of_solution** |
|---|---|
| **Paracetamol** | _1*gr* |
| **Dimethylacetamide (DMAC)** | *0.5gr* |
| **Metabisulphite Sodium** | *0.08gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 24

| **INGREDIENTS** | **Quantity for 100 ml of_solution** |
|---|---|
| **Paracetamol** | _1*gr* |
| **Dimethylacetamide (DMAC)** | *0.5gr* |
| **Edetate sodium** | *0.08gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 25

| **INGREDIENTS** | **Quantity for 100 ml of_solution** |
|---|---|
| **Paracetamol** | _1*gr* |
| **Alcohol 95°** | *5gr* |
| **Malic acid** | *0.08gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 26

| **INGREDIENTS** | **Quantity for 100 ml of_solution** |
|---|---|
| **Paracetamol** | _1*gr* |
| **Alcohol 95°** | *5gr* |
| **Metabisulphite sodium** | *0.07gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 27

| **INGREDIENTS** | **Quantity for 100 ml of_solution** |
|---|---|
| **Paracetamol** | _1*gr* |
| **Alcohol 95°** | *5gr* |
| **Edetate sodium** | *0.07gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 28

| **INGREDIENTS** | **Quantity for 100 ml of_solution** |
|---|---|
| **Paracetamol** | _1*gr* |
| **Alcohol 95°** | *5gr* |
| **BHA** | *0.002gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 29

| **INGREDIENTS** | **Quantity for 100 ml of_solution** |
|---|---|
| **Paracetamol** | _1*gr* |
| **Alcohol 95°** | *5gr* |
| **BHA** | *0.002gr* |
| **Malic acid** | *0.04gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 30

| **INGREDIENTS** | **Quantity for 100 ml of_solution** |
|---|---|
| **Paracetamol** | _1*gr* |
| **Dimethylacetamide (DMAC)** | *0.5gr* |
| **Alcohol 95°** | *2gr* |
| **Malic acid** | *0.02gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 31

| **INGREDIENTS** | **Quantity for 100 ml of_solution** |
|---|---|
| **Paracetamol** | _1*gr* |
| **Dimethylacetamide (DMAC)** | *0.5gr* |
| **Alcohol 95°** | *2gr* |
| **Metabisulphite sodium** | *0.08gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 32

| **INGREDIENTS** | **Quantity for 100 ml of_solution** |
|---|---|
| **Paracetamol** | _1*gr* |
| **Dimethylacetamide (DMAC)** | *0.5gr* |
| **Alcohol 95°** | *2gr* |
| **BHA** | *0.002gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 33

| **INGREDIENTS** | **Quantity for 100 ml of_solution** |
|---|---|
| **Paracetamol** | _1*gr* |
| **Dimethylacetamide (DMAC)** | *0.5gr* |
| **Alcohol 95°** | *2gr* |
| **Malic acid** | *0.04gr* |
| **BHA** | *0.001gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### Method Examples 34-41

The manufacturing process used for the examples 34 □41_is:
A. Dissolve Paracetamol using the solvent system, in hot water (70-75°C) or dissolve paracetamol in DMAC using also hot water (60°C)
B. Add the rest of water and cool the solution to room temperature (25° C)
C. Add the rest of excipients (antioxidant, tonicity agents)
D. Adjust the pH value by titration with HCL and/or NaOH 1N
E. Fill the solution into suitable containers

### EXAMPLE 34

| **INGREDIENTS** | **Quantity for 100 ml of_solution** |
|---|---|
| **Paracetamol** | _1*gr* |
| **Edetate sodium** | *0.05gr* |
| **Sodium chloride** | *0.66gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 35

| **INGREDIENTS** | **Quantity for 100 ml of_solution** |
|---|---|
| **Paracetamol** | _1*gr* |
| **Edetate sodium** | *0.1gr* |
| **Sodium chloride** | *0.66gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 36

| **INGREDIENTS** | **Quantity for 100 ml of_solution** |
|---|---|
| **Paracetamol** | _1*gr* |
| **Dimethylacetamide (DMAC)** | *0.5gr* |
| **Edetate sodium** | *0.05gr* |
| **Sodium chloride** | *0.49gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 37

| **INGREDIENTS** | **Quantity for 100 ml of_solution** |
|---|---|
| **Paracetamol** | _1*gr* |
| **Dimethylacetamide (DMAC)** | *0.5gr* |
| **Edetate sodium** | *0.1gr* |
| **Sodium chloride** | *0.49gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 38

| **INGREDIENTS** | **Quantity for 100 ml of_solution** |
|---|---|
| **Paracetamol** | _1*gr* |
| **Edetate sodium** | *0.05gr* |
| **Mannitol** | *0.85gr* |
| **sodium dihydrogen phosphate dihydrate** | *1.20gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 39

| **INGREDIENTS** | **Quantity for 100 ml of_solution** |
|---|---|
| **Paracetamol** | _1*gr* |
| **Dimethylacetamide (DMAC)** | *0.5g* |
| **Edetate sodium** | *0.05gr* |
| **Mannitol** | *1.10gr* |
| **sodium dihydrogen phosphate dihydrate** | *0.70gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 40

| **INGREDIENTS** | **Quantity for 100 ml of_solution** |
|---|---|
| **Paracetamol** | _1*gr* |
| **Dimethylacetamide (DMAC)** | *0.5g* |
| **Sodium metabisulphite** | *0.5gr* |
| **Mannitol** | *0.47gr* |
| **sodium dihydrogen phosphate dihydrate** | *0.35gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### EXAMPLE 41

| **INGREDIENTS** | **Quantity for 100 ml of_solution** |
|---|---|
| **Paracetamol** | _1*gr* |
| **Sodium metabisulphite** | *1.0gr* |
| **Mannitol** | *0.35gr* |
| **sodium dihydrogen phosphate dihydrate** | *0.42gr* |
| **Hydrochloric acid** | *qs to pH 5.50* |
| **Sodium hydroxide** | *qs to pH 5.50* |
| **Water for injection** | qs to 100 ml |
| **TOTAL VOLUME** | **100 ml** |

### PRE-STABILITY STUDIES

The preparations were subjected to stability studies. These studies indicate the relative stability of acetaminophen in the various formulations. In the result tables, NP indicates the test was not yet performed, and ND indicates the species were not detected.

### Experimental

Analytical column Thermo Hypersil BDS C18, 250 × 4.6 mm, 5 µm Part No. 28105-254630 or equivalent. References: Acetaminophen (paracetamol) reference material and 4-aminophenol reference material_of analytical quality were used as references. Reagents: HPLC-grade water, Methanol (MeOH), (HPLC grade), Sodium butanesulfonate, Formic acid, all of analytical quality. Chromatographic parameters: Mobile phase: 0.01M sodium butanesulphonate in a mixture of 0.4 volume of formic acid, 15 volumes of methanol and 85 volumes of water; Injection volume: 20 µl; Flow rate: 2.0 mL min-1; Column temperature: ambient; Run time(Assay): 10 min; Run time(Related substances): 45 min; Quantification wavelength: (a) assay 243 nm; (b) 4-aminophenol// unspecified impurities 272 nm.

### IDENTIFICATION OF IMPURITIES.

The identity of each impurity was determined by HPLC by the comparison of its Relative Retention Time (RRT) against the Retention Time of the main peak, which corresponds to the active material Paracetamol (acetaminophen). The main degradation product of acetaminophen is 4-aminophenol. NP: Not Performed, ND: Not Detected (below detection limit). All formulations yielded clear solutions during these studies

The following tables show the results after one month of storage at controlled temperature of normal (25 °C), 40 °C or 70 °C.

**Table 1: impurities in sulotion after 1 month. For examples 1-41.**

| **assay %** | | | | **Total related substances%** | | | **density (g/ml)** |
|---|---|---|---|---|---|---|---|
| | **conditions** | | | **conditions** | | | |
| **example** | **normal** | **40C** | **70C** | **normal** | **40C** | **70C** | |
| **1** | 104,7 | 104,7 | NP | ND | ND | NP | 1,113 |
| **2** | 105,9 | 105,9 | NP | ND | ND | NP | 1,133 |
| **3** | 105,7 | 105,7 | NP | ND | ND | NP | 1,031 |
| **4** | 106,6 | 107,3 | NP | 0,021 | 0,48 | NP | NP |
| **5** | 104 | 96,2 | NP | 0,019 | 0,1 | NP | NP |
| **6** | 92 | 91 | NP | 0,023 | 0,56 | NP | NP |
| **7** | NP | NP | NP | NP | NP | NP | NP |
| **8** | 95 | 95 | NP | 0,05 | 0,08 | NP | NP |
| **8.1** | 95 | 94 | NP | 0,048 | 0,078 | NP | NP |
| **8.2** | 101 | NP | NP | 0,048 | 0,08 | NP | NP |
| **8.3** | 101 | 100 | NP | 0,05 | 0,09 | NP | NP |
| **8.4** | 99 | 98 | NP | 0,06 | 0,07 | NP | NP |
| **8.5** | NP | NP | NP | NP | NP | NP | NP |
| **9** | NP | NP | NP | NP | NP | NP | NP |
| **9.1** | NP | NP | NP | NP | NP | NP | NP |
| **10** | NP | NP | NP | NP | NP | NP | NP |
| **11** | NP | NP | NP | NP | NP | NP | NP |
| **12** | NP | NP | NP | NP | NP | NP | NP |
| **13** | NP | NP | NP | NP | NP | NP | NP |
| **14** | NP | NP | NP | NP | NP | NP | NP |
| **15** | NP | NP | NP | NP | NP | NP | NP |
| **16** | NP | NP | NP | NP | NP | NP | NP |
| **17** | 99,5 | 98,6 | NP | NP | NP | NP | NP |
| **17.1** | 98,5 | 97,3 | NP | NP | NP | NP | NP |
| **17.2** | 97,6 | 97,5 | NP | NP | NP | NP | NP |
| **17.3** | 99,2 | 97,2 | NP | NP | NP | NP | NP |
| **17.4** | 98,6 | 95,6 | NP | NP | NP | NP | NP |
| **17.5** | 101,2 | 99,5 | NP | NP | NP | NP | NP |

| assay % | | | | Total related substances% | | | |
|---|---|---|---|---|---|---|---|
| | conditions | | | conditions | | | |
| example | normal | 40C | 70C | normal | 40C | 70C | |
| **18** | 110 | 109 | 105 | NP | NP | NP | |
| **18.1** | 102,3 | 101,5 | NP | NP | NP | NP | |
| **18.2** | 99,8 | 98,5 | NP | NP | NP | NP | |
| **18.3** | 96,3 | 94,3 | NP | NP | NP | NP | |
| **18.4** | 95,5 | 94,2 | NP | NP | NP | NP | |
| **18.5** | 94,8 | 93,2 | NP | NP | NP | NP | |
| **19** | 115 | 115 | NP | NP | NP | NP | |
| **20** | 113 | 112 | NP | NP | NP | NP | |
| **21** | 114 | 114 | NP | NP | NP | NP | |
| **22** | 105 | 103 | NP | NP | NP | NP | |
| **23** | 113 | 106 | NP | NP | NP | NP | |
| **24** | 100 | 106 | NP | NP | NP | NP | |
| **25** | 102 | 102 | NP | NP | NP | NP | |
| **26** | 103 | 104 | NP | NP | NP | NP | |
| **27** | 106 | 104 | NP | NP | NP | NP | |
| **28** | 103 | 98 | NP | NP | NP | NP | |
| **29** | 102 | 101 | NP | NP | NP | NP | |
| **30** | 102 | 102 | NP | NP | NP | NP | |
| **31** | 98 | 100 | NP | NP | NP | NP | |
| **32** | 105 | 105 | NP | NP | NP | NP | |
| **33** | 103 | 101 | NP | NP | NP | NP | |
| **34** | 101,5 | 101,4 | 100,5 | NP | NP | NP | |
| **35** | 102,6 | 102,4 | 100,3 | NP | NP | NP | |
| **36** | 102,5 | 102,6 | 101,3 | NP | NP | NP | |
| **37** | 101,5 | 101,6 | 100,3 | NP | NP | NP | |
| **38** | 103 | 103,9 | 103,4 | NP | NP | NP | |
| **39** | 101,8 | 102,8 | 101,2 | NP | NP | NP | |
| **40** | 103,7 | 103,9 | 102 | NP | NP | NP | |
| **41** | 100,9 | 102,2 | 101,7 | NP | NP | NP | |

**Table 2: pH as measured in solution after 1 month for examples 1- 17.5.**

| **pH** | | | |
|---|---|---|---|
| | **conditions** | | |
| **example** | **normal** | **40C** | **70C** |
| **1** | 5,5 | 5,5 | 5,5 |
| **2** | 5,5 | 5,5 | 5,5 |
| **3** | 5,5 | 5,5 | 5,5 |
| **4** | 5,5 | 5,5 | 5,5 |
| **5** | 5,5 | 5,5 | 5,5 |
| **6** | 5,5 | 5,5 | 5,5 |
| **7** | 5,5 | 5,5 | 5,5 |
| **8** | 5,4 | 5,4 | 5,4 |
| **8.1** | 5,4 | 5,4 | 5,4 |
| **8.2** | 5,4 | 5,4 | 5,4 |
| **8.3** | 5,4 | 5,4 | 5,4 |
| **8.4** | 5,4 | 5,4 | 5,4 |
| **8.5** | 5,4 | 5,4 | 5,4 |
| **9** | 4 | 4 | 4 |
| **9.1** | 4 | 4 | 4 |
| **10** | 5,5 | 5,5 | 5,5 |
| **11** | 5,5 | 5,5 | 5,5 |
| **12** | 5,5 | 5,5 | 5,5 |
| **13** | 5,5 | 5,5 | 5,5 |
| **14** | 5,5 | 5,5 | 5,5 |
| **15** | 5,5 | 5,5 | 5,5 |
| **16** | 5,5 | 5,5 | 5,5 |
| **17** | 5,5 | 5,5 | 5,5 |
| **17.1** | 5,5 | 5,5 | 5,5 |
| **17.2** | 5,5 | 5,5 | 5,5 |
| **17.3** | 5,5 | 5,5 | 5,5 |
| **17.4** | 5,5 | 5,5 | 5,5 |
| **17.5** | 5,5 | 5,5 | 5,5 |

**Table 3: pH and osmolarity after 1 month for examples 18-41. All solutions were clear.**

| | **pH** | | | **osmolarity** **(mOsm/kg)** |
|---|---|---|---|---|
| **example** | **normal** | **40C** | **70C** | |
| **18** | 4 | 4 | 4 | 88 |
| **18.1** | 5,5 | 5,5 | 5,5 | NP |
| **18.2** | 5,5 | 5,5 | 5,5 | NP |
| **18.3** | 5,5 | 5,5 | 5,5 | NP |
| **18.4** | 5,5 | 5,5 | 5,5 | NP |
| **18.5** | 5,5 | 5,5 | 5,5 | NP |
| **19** | 4,8 | 4,8 | 4,8 | 80 |
| **20** | 5,5 | 5,5 | 5,5 | 300 |
| **21** | 5,5 | 5,5 | 5,5 | 300 |
| **22** | 3,4 | 3,4 | 3,4 | 130 |
| **23** | 4,4 | 4,4 | 4,4 | 133 |
| **24** | 5 | 5 | 5 | 130 |
| **25** | 3,8 | 3,8 | 3,8 | 1115 |
| **26** | 3,8 | 3,8 | 3,8 | 1115 |
| **27** | 4,8 | 4,8 | 4,8 | 1118 |
| **28** | 6,3 | 6,3 | 6,3 | 1118 |
| **29** | 3,8 | 3,8 | 3,8 | 1118 |
| **30** | 3,8 | 3,8 | 3,8 | 550 |
| **31** | 3,8 | 3,8 | 3,8 | 550 |
| **32** | 5,6 | 5,6 | 5,6 | 550 |
| **33** | 3,8 | 3,8 | 3,8 | 550 |
| **34** | 5,5 | 5,5 | 5,5 | 292 |
| **35** | 5,3 | 5,3 | 5,3 | 295 |
| **36** | 5,3 | 5,3 | 5,3 | 294 |
| **37** | 5,4 | 5,4 | 5,4 | 295 |
| **38** | 5,4 | 5,4 | 5,4 | 285 |
| **39** | 5,4 | 5,4 | 5,4 | 320 |
| **40** | 5,4 | 5,4 | 5,4 | 298 |
| **41** | 5,5 | 5,5 | 5,5 | 350 |

**Table 4: impurities in examples 1- 17.5.**

| **4-aminophenol%** | | | | **total other related substances%** | | | **any other impurities%** | | |
|---|---|---|---|---|---|---|---|---|---|
| | **conditions** | | | **conditions** | | | **conditions** | | |
| **exa mpl e** | **normal** | **40C** | **70C** | **norm al** | **40C** | **70C** | **norm al** | **40C** | **70C** |
| **1** | NP | NP | NP | NP | NP | NP | NP | NP | NP |
| **2** | NP | NP | NP | NP | NP | NP | NP | NP | NP |
| **3** | NP | NP | NP | NP | NP | NP | NP | NP | NP |
| **4** | ND | ND | NP | NP | NP | NP | NP | NP | NP |
| **5** | 0,031 | 0,023 | NP | NP | NP | NP | NP | NP | NP |
| **6** | ND | ND | ND | NP | NP | NP | NP | NP | NP |
| **7** | NP | NP | NP | NP | NP | NP | NP | NP | NP |
| **8** | ND | ND | NP | NP | NP | NP | NP | NP | NP |
| **8.1** | ND | ND | NP | NP | NP | NP | NP | NP | NP |
| **8.2** | ND | ND | NP | NP | NP | NP | NP | NP | NP |
| **8.3** | ND | ND | NP | NP | NP | NP | NP | NP | NP |
| **8.4** | ND | ND | NP | NP | NP | NP | NP | NP | NP |
| **8.5** | NP | NP | NP | NP | NP | NP | NP | NP | NP |
| **9** | NP | NP | NP | NP | NP | NP | NP | NP | NP |
| **9.1** | NP | NP | NP | NP | NP | NP | NP | NP | NP |
| **10** | NP | NP | NP | NP | NP | NP | NP | NP | NP |
| **11** | NP | NP | NP | NP | NP | NP | NP | NP | NP |
| **12** | NP | NP | NP | NP | NP | NP | NP | NP | NP |
| **13** | NP | NP | NP | NP | NP | NP | NP | NP | NP |
| **14** | NP | NP | NP | NP | NP | NP | NP | NP | NP |
| **15** | NP | NP | NP | NP | NP | NP | NP | NP | NP |
| **16** | NP | NP | NP | NP | NP | NP | NP | NP | NP |
| **17** | NP | NP | NP | NP | NP | NP | NP | NP | NP |
| **17.1** | NP | NP | NP | NP | NP | NP | NP | NP | NP |
| **17.2** | NP | NP | NP | NP | NP | NP | NP | NP | NP |
| **17.3** | NP | NP | NP | NP | NP | NP | NP | NP | NP |
| **17.4** | NP | NP | NP | NP | NP | NP | NP | NP | NP |
| **17.5** | NP | NP | NP | NP | NP | NP | NP | NP | NP |

**Table 5: Impurities after 1 month in examples 18-41.**

| **4-aminophenol%** | | | | **total other related substances%** | | | **any other impurities%** | | |
|---|---|---|---|---|---|---|---|---|---|
| | **conditions** | | | **conditions** | | | **conditions** | | |
| **example** | **norm al** | **40C** | **70C** | **normal** | **40C** | **70C** | **normal** | **40C** | **70C** |
| **18** | 0,69 | ND | NP | NP | NP | NP | NP | NP | NP |
| **18.1** | 0,7 | ND | NP | NP | NP | NP | NP | NP | NP |
| **18.2** | 0,8 | ND | NP | NP | NP | NP | NP | NP | NP |
| **18.3** | 0,45 | ND | NP | NP | NP | NP | NP | NP | NP |
| **18.4** | 1,1 | ND | NP | NP | NP | NP | NP | NP | NP |
| **18.5** | 1 | ND | NP | NP | NP | NP | NP | NP | NP |
| **19** | 0,01 | ND | NP | ND | ND | NP | NP | NP | NP |
| **20** | 0,01 | 0,01 | NP | 0,28 | 0,36 | NP | NP | NP | NP |
| **21** | 0,01 | 0,01 | NP | 0,52 | 0,55 | NP | NP | NP | NP |
| **22** | 0,21 | 0,18 | NP | ND | 0,31 | NP | NP | NP | NP |
| **23** | 0,03 | 0,08 | NP | ND | ND | NP | NP | NP | NP |
| **24** | 0,01 | ND | NP | ND | ND | NP | NP | NP | NP |
| **25** | 0,12 | 0,01 | NP | 0,15 | 0,41 | NP | NP | NP | NP |
| **26** | 0,07 | 0,43 | NP | ND | ND | NP | NP | NP | NP |
| **27** | ND | ND | NP | ND | ND | NP | NP | NP | NP |
| **28** | ND | ND | NP | ND | ND | NP | NP | NP | NP |
| **29** | 0,12 | 0,07 | NP | 0,12 | 0,57 | NP | NP | NP | NP |
| **30** | 0,36 | 0,19 | NP | ND | 0,43 | NP | NP | NP | NP |
| **31** | 0,07 | 0,31 | NP | ND | ND | NP | NP | NP | NP |
| **32** | ND | ND | NP | ND | ND | NP | NP | NP | NP |
| **33** | 0,14 | 0,06 | NP | 0,16 | 0,58 | NP | NP | NP | NP |
| **34** | ND | ND | ND | 0,02 | 0,1 | 1,4 | 0,01 | 0,05 | 0,81 |
| **35** | ND | ND | ND | 0,02 | 0,09 | 1,4 | 0,01 | 0,04 | 0,87 |
| **36** | ND | ND | ND | 0,02 | 0,09 | 1,4 | 0,01 | 0,05 | 0,8 |
| **37** | ND | ND | ND | 0,02 | 0,1 | 1,5 | 0,01 | 0,06 | 0,9 |
| **38** | ND | ND | ND | 0,06 | 0,12 | 1,2 | 0,02 | 0,05 | 0,55 |
| **39** | ND | ND | ND | 0,05 | 0,12 | 1 | 0,02 | 0,06 | 0,59 |
| **40** | 0,15 | 0,4 | 1,5 | 0,2 | 0,46 | 1,6 | 0,02 | 0,02 | 0,02 |
| **41** | 0,7 | 1,6 | 6,6 | 0,75 | 1,7 | 6,6 | 0,01 | 0,02 | 0,02 |

The following tables show impurities and pH in the examples 34-41 after 2 months of storages under controlled conditions. All formulations were clear solutions.

**Table 6: impurities after 2 months of storage for formulations 34-41.**

| **2_MONTHS FORCED STUDIES** | | | | | |
|---|---|---|---|---|---|
| **assay%** | | | **Total related substances%** | | **density (g/ml)** |
| | **conditions** | | **conditions** | | |
| **example** | **40C** | **70C** | **40C** | **70C** | |
| **34** | 99,2 | 96,8 | NP | NP | NP |
| **35** | 101,4 | 97,5 | NP | NP | NP |
| **36** | 100,1 | 96,5 | NP | NP | NP |
| **37** | 99,1 | 95,5 | NP | NP | NP |
| **38** | 99,7 | 95,6 | NP | NP | NP |
| **39** | 99 | 93,2 | NP | NP | NP |
| **40** | 97 | 92,7 | NP | NP | NP |
| **41** | 93,2 | 67,5 | NP | NP | NP |

**Table 7: pH and osmolarity of formulations 34-41 after 2 months of storage. All formulations were clear solutions.**

| | **pH** | | **osmolarity** **(mOsm/kg)** |
|---|---|---|---|
| **example** | **40C** | **70C** | |
| **34** | 5,3 | 5,3 | 290 |
| **35** | 5,3 | 5,3 | 290 |
| **36** | 5,4 | 5,4 | 292 |
| **37** | 5,3 | 5,3 | 295 |
| **38** | 5,5 | 5,5 | 286 |
| **39** | 5,5 | 5,5 | 320 |
| **40** | 5,4 | 5,4 | 296 |
| **41** | 5,4 | 5,4 | 350 |

**Table 8: impurities in formulations 34-41 after 2 months of storage.**

| **4-aminophenol%** | | | **total other related substances%** | | **any other impurities%** | |
|---|---|---|---|---|---|---|
| | **conditions** | | **conditions** | | **conditions** | |
| **example** | **40C** | **70C** | **40C** | **70C** | **40C** | **70C** |
| **34** | 0,004 | 0,007 | 0,22 | 2,4 | 0,13 | 1 |
| **35** | 0,002 | 0,008 | 0,21 | 2,2 | 0,13 | 1 |
| **36** | 0,003 | 0,008 | 0,2 | 2,7 | 0,12 | 1 |
| **37** | 0,004 | 0,009 | 0,26 | 2,8 | 0,15 | 1 |
| **38** | ND | ND | 0,23 | 2 | 0,1 | 0,72 |
| **39** | ND | ND | 0,21 | 1,9 | 0,11 | 0,85 |
| **40** | 0,42 | 1,1 | 0,48 | 1,2 | 0,02 | 0,05 |
| **41** | 2,1 | 4,6 | 4,6 | 20 | 0,03 | 0,01 |

### Perfalgan

For comparison, the stability tests were also performed on the commercially available PERFALGAN®10 MG/ML formulation, comprising 10 mg/ml of acetaminophen. NP: Not Performed, ND: Not Detected (below detection limit)

**Table 9: impurities, pH and osmolarity of Perfalgan after 1 month of storage under controlled conditions.**

| **conditions** | | | **conditions** | | | **Density** | | |
|---|---|---|---|---|---|---|---|---|
| **normal** | **40C** | **70C** | **normal** | **40C** | **70C** | **(g/ml)** | | |
| 98,8 | 98,6 | 97,1 | 0,03 | 0,03 | 0,4 | NP | | |

| **appearance** | | | **pH** | | | | | |
|---|---|---|---|---|---|---|---|---|
| **conditions** | | | **conditions** | | | | | |
| **normal** | **40C** | **70C** | **normal** | **40C** | **70C** | **osmolarity (mOsm/kg)** | | |
| clear solution | | | 5,7 | 5,6 | 5,4 | 296 | | |

| **4-aminophenol%** | | | **total other related substances%** | | | **any other impurities%** | | |
|---|---|---|---|---|---|---|---|---|
| **conditions** | | | **conditions** | | | **conditions** | | |
| **normal** | **40C** | **70C** | **normal** | **40C** | **70C** | **normal** | **40C** | **70C** |
| 0,02 | 0,02 | 0,22 | 0,01 | 0,01 | 0,02 | 0,01 | 0,01 | 0,11 |

**Table 9: impurities, pH and osmolarity of Perfalgan after 2 months of storage under controlled conditions.**

| **conditions** | | | **conditions** | | | **density (g/ml)** | | |
|---|---|---|---|---|---|---|---|---|
| **normal** | **40C** | **70C** | **normal** | **40C** | **70C** | | | |
| | 97 | 96,6 | | 0,08 | 0,33 | | NP | |

| **appearance** | | | **pH** | | | | | |
|---|---|---|---|---|---|---|---|---|
| **conditions** | | | **conditions** | | | | | |
| **normal** | **40C** | **70C** | **normal** | **40C** | **70C** | **osmolarity (mOsm/kg)** | | |
| clear solution | | | 5,7 | 5,5 | 5,4 | 296 | | |

| **4-aminophenol%** | | | **total other related substances%** | | | **any other impurities%** | | |
|---|---|---|---|---|---|---|---|---|
| **conditions** | | | **conditions** | | | **conditions** | | |
| **normal** | **40C** | **70C** | **normal** | **40C** | **70C** | **normal** | **40C** | **70C** |
| | 0,03 | 0,36 | | NP | NP | | 0,04 | 0,31 |

## Claims

1. Liquid composition suitable for parenteral administration, comprising at least 1 mg/ml of acetaminophen, preferably at least 10 mg/ml, most preferably at least 100 mg/ml, and at least one pharmaceutically acceptable excipient.

2. Liquid composition according to claim 1, comprising at least 1 mg/ml of acetaminophen, and at least one pharmaceutically acceptable chelating agent.

3. Composition according to claim 2, wherein the chelating agent comprises at least one water-soluble chelating agent selected from the group consisting of malic acid, fumaric acid, tartaric acid, edetic acid, dipotassium edetate; disodium edetate, calcium disodium, monosodium edetate; trisodium edetate, ethylenediamine tetraacetic acid (EDTA), citric acid, anhydrous citric acid, sodium citrate dihydrate, maltol and/or ethyl maltol, and/or pharmaceutically acceptable derivatives thereof.

4. Composition according to claim 3, wherein the at least one water-soluble chelating agent comprises ethylenediamine tetraacetic acid or a pharmaceutically acceptable salt thereof.

5. Composition according to any of the preceding claims, wherein the composition also comprises at least one tonicity agent selected from the group consisting of mannitol, sodium chloride and phosphate salts.

6. Composition according to claim 5, wherein the composition comprises a combination of mannitol and disodium phosphate.

7. Composition according to any of the preceding claims, wherein the composition also comprises at least one acetaminophen solubilising agent.

8. Composition according to claim 7, wherein the solubilising agent is selected from the group consisting of dimethylacetamide, glycerine, glycofurol, povidone, ethanol alcohol (95%) or admixtures thereof.

9. Composition according to claim 7 or 8, wherein the solubilising agent comprises dimethylacetamide.

10. Composition according to any of the claims 7-9, wherein the solubilising agent comprises dimethylacetamide in quantity of at least 5 mg/ml of the total solution, preferably at least 10 mg/ml.

11. Composition according to any of the claims 7-10, wherein the solubilising agent comprises dimethylacetamide in a ratio of at least 1:1 w/w with respect to acetaminophen, preferably in the range of 1:1 □1:3.

12. Composition according to any of the preceding claims, wherein the composition also comprises at least one antioxidant.

13. Composition according to any of the preceding claims, wherein the composition is an aqueous solution having a pH between 5 and 6, preferably having a pH of 5.4-5.6.

14. Composition according to any of the preceding claims, wherein the composition is suitable for intravenous injection and/or intramuscular injection.

15. Method for the preparation of a composition according to any of the preceding claims, comprising the steps of:
- dissolving at least 1 mg/ml of acetaminophen, preferably at least 10 mg/ml, most preferably at least 100 mg/ml or a derivative thereof in a solvent, preferably water,
- and dissolving at least one pharmaceutically acceptable excipient in the solvent.
